Europäisches Patentamt

European Patent Office

Office européen des brevets

(11) Numéro de publication : **0 635 267 A1**

## (12) DEMANDE DE BREVET EUROPEEN

(21) Numéro de dépôt : **94420212.6**

(22) Date de dépôt : **22.07.94**

(51) Int. Cl.[6] : **A61K 31/20**

(30) Priorité : **23.07.93 FR 9309313**

(43) Date de publication de la demande :
**25.01.95 Bulletin 95/04**

(84) Etats contractants désignés :
**AT BE CH DE DK ES FR GB GR IE IT LI LU MC
NL PT SE**

(71) Demandeur : **Doutreleau, Jean-Claude
Masia Can Guardia,
Salve Regina, S/N
E-08187 Sta. Eulalia de Roncana (ES)**

(72) Inventeur : **Doutreleau, Jean-Claude
Masia Can Guardia,
Salve Regina, S/N
E-08187 Sta. Eulalia de Roncana (ES)**

(74) Mandataire : **Monnier, Guy et al
Cabinet Monnier
142-150 Cours Lafayette
B.P. 3058
F-69392 Lyon Cédex 03 (FR)**

(54) **Compositions à base d'acides gras douées de propriétés anti-inflammatoires.**

(57) Ces compositions renferment, à titre de principe actif principal, dans un support pharmaceutiquement acceptable, un ou plusieurs sels d'un ou plusieurs acides gras présentant la formule générale :
$$CH_3 - C_x..H_y..O_z - COO^- \; X^+$$
dans laquelle $x = 2$ à $30$, $y = 2$ à $60$, $z = 0$ à $6$ et $X^+$ est le cation de de la base utilisée (de préférence soude ou potasse)

Les acides gras sont choisis parmi ceux dérivant de substances naturelles, et notamment des huiles et graisses d'origine animale ou végétale. Le ou les sels d'acides gras peuvent être associés à un ou plusieurs anti-inflammatoires stéroïdien ou non stéroïdien.

Ces compositions sont prévues pour application par voie parentérale, par voie orale, par voie rectale, par voie diathélique, ainsi que par voie dermique et intradermique.

EP 0 635 267 A1

Jouve, 18, rue Saint-Denis, 75001 PARIS

La présente invention concerne des compositions à base d'acides gras, douées de propriétés antiinflammatoires particulièrement destinées au traitement des états inflammatoires chez l'homme et l'animal.

On peut distinguer deux grands groupes de substances possédant des propriétés anti-inflammatoires, substances que l'on peut différencier tant en fonction de leur structure chimique que de leur mécanisme d'action et de leur utilisation thérapeutique chez l'homme ou l'animal.

Le premier groupe comporte les anti-inflammatoires stéroïdiens ou glucocorticoïdes, qu'ils soient naturels ou semi-synthétiques.

Ces anti-inflammatoires sont très largement utilisés dans de nombreuses indications mais cette utilisation entraîne toujours des risques et des inconvénients graves; il peut s'agir d'action pro-infectieuse (d'autant plus importante que l'action anti-inflammatoire est forte) en raison des propriétés immunodépressives rendant obligatoire une couverture antibiotique ; leur utilisation est contre-indiquée lors d'infections virales et lors des vaccinations.

Ces substances exercent aussi des effets métaboliques : ostéoporose, hyperglycémie, rétention d'eau, dépôts graisseux anormaux, diminution de la diurèse ; des effets hypophysaires : inhibition durable de l'axe hypothalamo-hypophysaire interdisant l'arrêt brutal de la médication ; des effets physiologiques (atonie générale, fatigabilité), hormonaux (syndrome de Cushing), digestifs, rénaux...

Un autre groupe de substances est constitué par les anti-inflammatoires non stéroïdiens dont le mode d'action repose essentiellement sur l'inhibition de la synthèse des prostaglandines, ce qui explique leurs actions anti-inflammatoire, analgésique et antipyrétique.

Il s'agit de produits salicylés, pyrazolés, de certains enzymes, ainsi que de dérivés indoliques, anthraniliques, aryl-propioniques, diméthylsulfoxyde, sulfonanilide...

Les composés de ce groupe présentent, eux aussi, des effets secondaires importants parmi lesquels on peut citer ; irritation gastro-intestinale et ulcérations, néphropathies, hépatotoxicité cholestatique et parenchymateuse, diverses altérations sanguines, méthémoglobinémie, hypoprothrombinémie...

L'inventeur a déterminé, de façon surprenante, que certains sels d'acides gras permettaient d'obtenir des compositions douées de propriétés anti-inflammatoires mais qui ne présentaient, aux doses usuelles, aucun des effets secondaires mentionnés ci-avant, permettant ainsi une utilisation très large et très prolongée.

Les compositions douées de propriétés anti-inflammatoires selon l'invention renferment, à titre de principe actif principal, dans un support pharmaceutiquement acceptable, un ou plusieurs sels d'un ou plusieurs acides gras présentant la formule générale :

$$CH_3 - C_x..H_y..O_z - COO^- \; X^+$$

dans laquelle x = 2 à 30, y = 2 à 60, z = 0 à 6 et $X^+$ est le cation de la base utilisée.

Le ou les acides gras sont choisis de préférence parmi ceux contenus dans les substances naturelles, et notamment les huiles et graisses d'origine animale ou végétale.

Selon un mode de réalisation préféré de l'invention, la base est choisie parmi la soude ou la potasse, sans que cela constitue toutefois une limitation à l'invention.

Selon un mode de réalisation de l'invention, les acides gras sont saturés et présentent un nombre pair ou impair d'atomes de carbone. Ils peuvent être ramifiés et présentent alors les formes iso et antéiso.

Selon un autre mode de réalisation de l'invention, les acides gras sont monoéniques et sont choisis pour un même acide, parmi les différents isomères, notamment les acides monoéniques à double liaison en 9, les acides monoéniques à double liaison en n-9 et les acides monoéniques à double liaison cis ou trans.

Les acides gras peuvent être polyéniques et, pour un même acide polyénique, être choisis parmi les différents isomères, notamment les acides polyéniques à méthylènes centraux (où chaque double liaison est séparée de la suivante par un seul méthylène), les acides polyéniques à doubles liaisons en forme cis et les acides polyéniques à doubles liaisons conjuguées ou écartées.

Ils peuvent être choisis parmi d'autres acides gras comme les acides acétyléniques et les acides cycliques.

Selon un autre mode de réalisation, les acides gras ont des fonctions secondaires, notamment les hydroxyacides et les cétoacides.

Les acides gras sont de préférence choisis parmi les substances naturelles, et notamment les huiles et graisses d'origine animale ou végétale

Ces acides sont saponifiés selon la réaction :

Triglycérides + base → Sels d'acides gras + glycérol.

Les compositions de sels d'acides gras selon l'invention sont administrées à l'homme ou à l'animal par toute voie classique.

Quand les compositions sont administrées par voie parentérale, les sels d'acides gras sont tout d'abord dissous dans de l'eau pour obtenir des préparations injectables à des concentrations variant de 2,5 g/l à 120 g/l, les concentrations usuelles étant comprises entre 10g/l et 40 g/l.

Elles peuvent également être administrées par voie rectale, par voie orale ou par voie dermique ou intradermique, la posologie étant alors ajustée en conséquence.

Les compositions selon l'invention peuvent également être administrées à la vache et à la chèvre par voie diathélique.

Les exemples suivants, permettront de mieux comprendre l'invention et tout l'intérêt qu'elle présente, sans nullement la limiter.

Dans tous les exemples, l'effet anti-inflammatoire obtenu est mis en évidence par le test pharmacologique "Oedéme à la carragénine chez le rat" selon la technique décrite par Winter C.A. et al in Proc. Soc. Exp. Biol. (N.Y.), 1962 111, 544. Les animaux reçoivent 3 heures et 1 heure avant l'injection intraplantaire de carragénine la composition à tester par administration intrapéritonéale, à la dose de 0,9 ml/250 g de poids vif.

L'oedème est mesuré aux temps : $t_0$, $t_{1h}$, $t_{2h}$, $t_{3h}$, $t_{4h}$ après l'injection de carragénine.

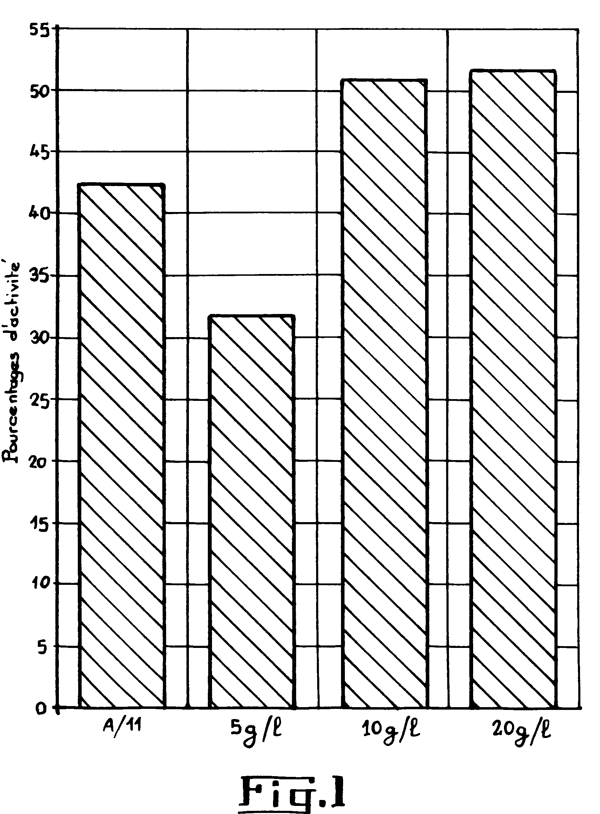

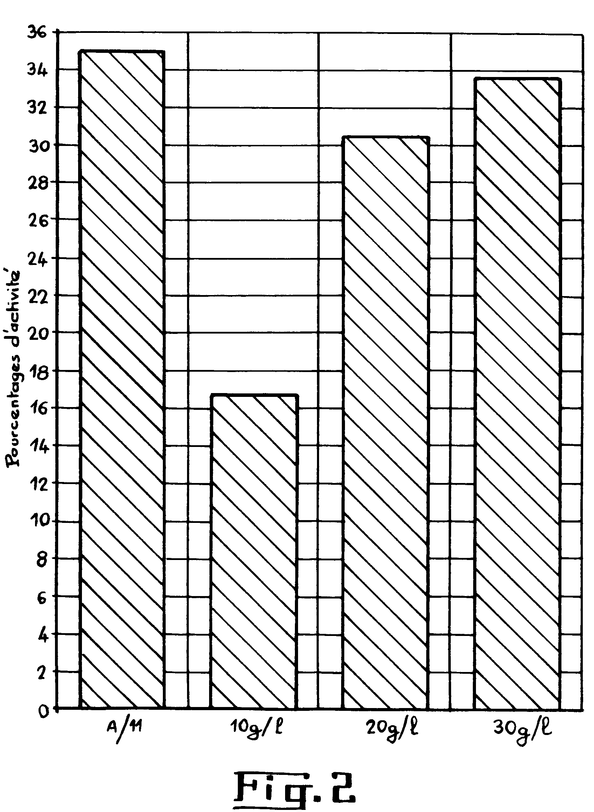

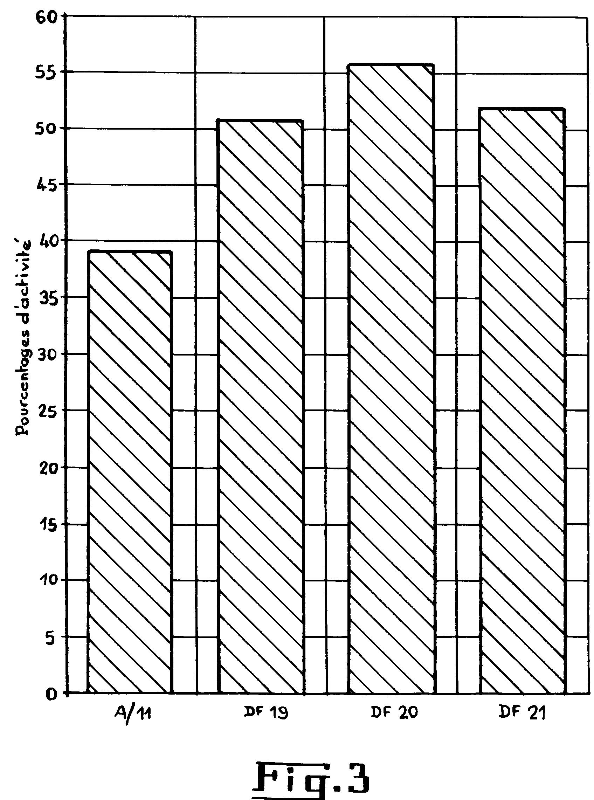

Les résultats rassemblés sur les tableaux ci-joints permettent de délimiter une aire sur le graphique oedème-temps ; des graphiques à barre, explicitant certains de ces résultats, sont représentés au dessin schématique annexé dans lequel :

Le graphique-barre 1 illustre les essais réalisés avec du suif et de l'huile de coprah, seuls ou en mélange

Le graphique-barre 2 illustre les essais réalisés avec de l'huile de pépins de raisins

Le graphique-barre 3 illustre les essais réalisés avec de l'huile de coprah.

Sur ces graphiques, le pourcentage d'activité a été porté en ordonnées, et le produit-étalon et les différentes concentrations du ou des produits examinés en abscisses.

L'activité des compositions selon l'invention est définie comparativement à des mesures de populations-témoins. Le produit de référence (Al 11, 12 ou 13) est une molécule codée de composition parfaitement connue et reproductible qui sert d'étalon et permet de vérifier la validité statistique des tests.

Exemple 1 - Corps gras de type laurique : huile de Coprah ou palmiste

On a testé une solution aqueuse de sels sodiques d'acides gras d'huile de Coprah à des concentrations variant de 5 à 20 g/l. Les résultats, rassemblés sur le tableau I, montrent que le pourcentage d'activité varie de 30 à plus de 50 %.

L'utilisation de solutions plus concentrées exige une adaptation de la posologie.

TABLEAU 1

| LOT | % d'oedème | | | | Aire sous la courbe | % d'activité |
|---|---|---|---|---|---|---|
| | t1 | t2 | t3 | t4 | | |
| Témoin | 45,8 | 58,2 | 55,9 | 48,7 | 184,7 ± 37,9 | |
| Al 11 | 23,3 | 31,9 | 35,7 | 28,1 | 106,2 ± 56,6 | 42,5 |
| 5 g/l | 27,4 | 38,9 | 42,1 | 33,8 | 125,8 ± 19,8 | 31,9 |
| 10 g/l | 16,5 | 28,3 | 33,4 | 23,6 | 90,5 ± 21,3 | 51,0 |
| 20 g/l | 19,1 | 28,4 | 29,9 | 23,2 | 89,2 ± 17,7 | 51,8 |

Les résultats de l'exemple 1 sont par ailleurs illustrés par le graphique 1.

Exemple 2 - Corps gras constitués d'acides gras à chaînes carbonées moyennes : huile de palme, de cacao, Illipé de Bornéo, beurre de Karité...

On a testé une solution aqueuse de sels sodiques d'acides gras d'huile de palme à des concentrations variant de 10 à 30 g/l. Les résultats, rassemblés sur le tableau 2 montrent que le pourcentage d'activité varie de 25 à près de 40 %. L'utilisation de solutions plus concentrées exige une adaptation de la posologie.

TABLEAU 2

| LOT | % d'oedème | | | | Aire sous la courbe | % d'activité |
|---|---|---|---|---|---|---|
| | t1 | t2 | t3 | t4 | | |
| Témoin | 37,4 | 49,4 | 50,4 | 40,6 | 157,6 ±25,9 | |
| Al 11 | 17,0 | 23,5 | 26,3 | 21,2 | 77,4 ± 22,5 | 50,9 |
| 10 g/l | 27,2 | 39,3 | 36,7 | 27,0 | 117,7 ± 42,4 | 25,3 |
| 20 g/l | 25,7 | 37,1 | 35,9 | 27,5 | 112,3 ± 22,5 | 28,7 |
| 30 g/l | 23,6 | 33,7 | 30,4 | 22,0 | 99,0 ± 34,1 | 37,2 |

Exemple 3 - Corps gras d'origine animale : suif, saindoux, graisses d'os, de volaille....

On a testé une solution aqueuse de sels sodiques d'acides gras de suif à des concentrations variant de 10 à 30 g/l. Les résultats, rassemblés sur le tableau 3 montrent que le pourcentage d'activité varie de 35 à 60 %. L'utilisation de solutions plus concentrées exige une adaptation de la posologie.

TABLEAU 3

| LOT | % d'oedème | | | | Aire sous la courbe | % d'activité |
|---|---|---|---|---|---|---|
| | t1 | t2 | t3 | t4 | | |
| Témoin | 43,5 | 57,8 | 64,3 | 56,3 | 193,6 ±36,1 | |
| Al 11 | 17,2 | 27,2 | 24,7 | 19,6 | 79,2 ± 37,5 | 59,1 |
| 10 g/l | 26,7 | 38,1 | 42,7 | 35,1 | 124,7 ± 34,8 | 35,6 |
| 20 g/l | 19,0 | 29,9 | 34,1 | 28,0 | 95,8 ± 35,7 | 50,5 |
| 30 g/l | 16,3 | 25,9 | 26,4 | 20,9 | 79,1 ± 16,8 | 59,1 |

Exemple 4 - Corps gras d'origine animale marine : huile de saumon, de hareng, d'anchois, de sardine, de Menhaden...

On a testé une solution aqueuse de sels sodiques d'acides gras d'huile de saumon à des concentrations variant de 10 à 30 g/l. Les résultats, rassemblés sur le tableau 4 montrent que le pourcentage d'activité varie de 18 à 43 %. L'utilisation de solutions plus concentrées exige une adaptation de la posologie.

TABLEAU 4

| LOT | % d'oedème | | | | Aire sous la courbe | % d'activité |
|---|---|---|---|---|---|---|
| | t1 | t2 | t3 | t4 | | |
| Témoin | 40,8 | 51,8 | 55,3 | 45,1 | 170,7 ± 28,7 | |
| Al 11 | 17,5 | 32,0 | 34,0 | 24,9 | 95,5 ± 40,5 | 44,1 |
| 10 g/l | 31,9 | 43,9 | 44,4 | 35,6 | 138,9 ± 25,3 | 18,6 |
| 20 g/l | 25,4 | 35,6 | 38,5 | 30,3 | 114,6 ± 24,3 | 32,9 |
| 30 g/l | 20,2 | 29,0 | 34,1 | 27,4 | 97,2 ± 17,2 | 43,1 |

Exemple 5 - Corps gras d'origine végétale à acides gras monoéniques : huile d'olive, d'amande, de noisette, d'arachide, de thé, de riz, d'avocat...

On a testé une solution aqueuse de sels sodiques d'acides gras d'huile d'olive à des concentrations variant de 2,5 à 120 g/l. Les résultats, rassemblés sur le tableau 5, montrent que le pourcentage d'activité varie de 13 à 50 %. L'utilisation de solutions plus concentrées exige une adaptation de la posologie.

TABLEAU 5

| LOT | % d'oedème | | | | Aire sous la courbe | % d'activité |
|---|---|---|---|---|---|---|
| | t1 | t2 | t3 | t4 | | |
| Témoin | 32,7 | 43,5 | 48,8 | 38,7 | $144,1 \pm 23,8$ | |
| Al 11 | 19,1 | 28,0 | 33,1 | 25,2 | $93,3 \pm 30,6$ | 35,3 |
| 2,5 g/l | 28,2 | 37,8 | 41,9 | 32,7 | $124,9 \pm 26,1$ | 13,3 |
| 5 g/l | 25,3 | 37,4 | 37,3 | 29,4 | $115,6 \pm 22,8$ | 19,8 |
| 120 g/l | 12,5 | 23,8 | 25,4 | 20,3 | $71,5 \pm 27,5$ | 50,4 |

Exemple 6 - Corps gras d'origine végétale à acides gras diéniques : huile de pépins de raisin, de maïs, de coton, de sésame, de tournesol, de soja, d'oeillette, de cameline,de carthame, de noix, de germe de blé...

On a testé une solution aqueuse de sels sodiques d'acides gras d'huile de pépins de raisin à des concentrations de 10, 20 et 30 g/l. Les résultats rassemblés sur le graphique 2 montrent que le pourcentage d'activité varie de 16 à 34 %. L'utilisation de solutions plus concentrées exige une adaptation de la posologie.

Exemple 7 - Corps gras d'origine végétale à acides gras triéniques : huile de lin, de Perilla, de Bois de Chine, d'Oïticica...

On a testé une solution aqueuse de sels sodiques d'acides gras d'huile de lin à des concentrations variant de 2,5 à 120 g/l. Les résultats rassemblés sur le tableau 6 montrent que le pourcentage d'activité varie de 22 à 86 %. L'utilisation de solutions plus concentrées exige une adaptation de la posologie.

TABLEAU 6

| LOT | % d'oedème | | | | Aire sous la courbe | | % d'activité |
|---|---|---|---|---|---|---|---|
| | t1 | t2 | t3 | t4 | m | CV | |
| Témoin | 48,9 | 63,3 | 65,4 | 54,0 | 205,6 | 0,062 | |
| Al 12 | 29,0 | 37,5 | 37,7 | 28,8 | 118,5 | 0,100 | 42,4 |
| 2,5 g/l | 36,7 | 51,9 | 51,4 | 40,8 | 160,2 | 0,063 | 22,1 |
| 30 g/l | 20,2 | 30,1 | 31,2 | 21,5 | 92,6 | 0,142 | 55,0 |
| 120 g/l | 5,4 | 9,3 | 11,0 | 7,1 | 89,7 | 0,173 | 85,6 |

Les compositions selon l'invention peuvent également être obtenues à partir de mélanges de corps gras de différentes origines.

Exemple 8 - Mélanges de corps gras

On a testé des solutions aqueuses de sels sodiques d'acides gras de 20 g/l de suif (ref. DF 19), de 20 g/l d'huile de coprah (ref DF 20) et d'un mélange à 30 g/l de 2/3 de suif et de 1/3 d'huile de coprah (ref. DF 21). Les résultats rassemblés sur le tableau 7 et le graphique 3 montrent que le pourcentage d'activité varie de 50 à 56 %.

TABLEAU 7

| LOT | % d'oedème | | | | Aire sous la courbe | % d'activité |
|------|------|------|------|------|------|------|
| | t1 | t2 | t3 | t4 | | |
| Témoin | 40,8 | 52,2 | 59,4 | 52,4 | 178,9 ± 42,9 | |
| Al 11 | 23,3 | 37,0 | 35,5 | 27,8 | 109,0 ± 44,6 | 39,1 |
| DF 19 | 17,8 | 30,1 | 30,2 | 19,1 | 87,8 ± 24,0 | 50,9 |
| DF 20 | 17,7 | 25,6 | 26,0 | 19,3 | 79,00 ± 19,6 | 55,8 |
| DF 21 | 18,6 | 27,7 | 28,2 | 21,9 | 85,8 ± 32,2 | 52,0 |

Des essais similaires effectués sur une solution à 30 g/l de sels sodiques d'acides gras obtenue par mélange de 50 % de Suif, 25 % d'huile de Coprah et 25 % d'huile d'olive montrent un pourcentage d'activité de 43 %.

On a examiné par ailleurs des compositions obtenues à partir d'acides gras classiques.

Exemple 9 - Acides gras impairs

On a testé une solution aqueuse de sels sodiques d'un acide gras impair, l'acide undecanoïque, à des concentrations variant de 2,5 à 10 g/l Les résultats rassemblés sur le tableau 8 montrent que le pourcentage d'activité varie de 5 à 25%. L'utilisation de solutions plus concentrées exige une adaptation de la posologie.

TABLEAU 8

| LOT | % d'oedème | | | | Aire sous la courbe | | % d'activité |
|------|------|------|------|------|------|------|------|
| | t1 | t2 | t3 | t4 | m | CV | |
| Témoin | 43,3 | 58,2 | 67,8 | 59,5 | 199,1 | 0,050 | |
| Al 12 | 21,7 | 30,3 | 32,8 | 25,4 | 97,5 | 0,159 | 51,0 |
| 2,5 g/l | 45,8 | 58,2 | 59,7 | 49,4 | 188,3 | 0,070 | 5,4 |
| 10 g/l | 31,6 | 46,2 | 49,9 | 42,7 | 149,5 | 0,096 | 24,9 |

Exemple 10 - Acides gras monoéniques isomères trans

On a testé une solution aqueuse de sels sodiques d'acide élaïdique, à des concentrations variant de 10 à 60 g/l Les résultats rassemblés sur le tableau 9 montrent que le pourcentage d'activité varie de 28 à 68 %.

TABLEAU 9

| LOT | % d'oedème | | | | Aire sous la courbe | | % d'activité |
|---|---|---|---|---|---|---|---|
| | t1 | t2 | t3 | t4 | m | CV | |
| Témoin | 32,4 | 52,4 | 56,0 | 44,1 | 163,4 | 0,063 | |
| Al 12 | 17,3 | 21,4 | 27,5 | 17,8 | 74,6 | 0,110 | 54,3 |
| 10 g/l | 17,3 | 39,3 | 42,6 | 35,6 | 117,6 | 0,100 | 28,0 |
| 30 g/l | 10,0 | 15,3 | 20,3 | 14,9 | 53,1 | 0,147 | 67,5 |
| 60g/l | 12,8 | 18,3 | 20,9 | 12,3 | 58,3 | 0,105 | 64,3 |

## Exemple 11 - Acides gras ramifiés

On a testé une solution aqueuse de sels sodiques d'acide isostéarique, à des concentrations variant de 2,5 à 100 g/l Les résultats rassemblés sur le tableau 10 montrent que le pourcentage d'activité varie de 1 à 50 %.

TABLEAU 10

| LOT | % d'oedème | | | | Aire sous la courbe | | % d'activité |
|---|---|---|---|---|---|---|---|
| | t1 | t2 | t3 | t4 | m | CV | |
| Témoin | 41,1 | 55,4 | 56,2 | 43,7 | 174,6 | 0,056 | |
| Al 12 | 18,3 | 26,9 | 29,0 | 20,4 | 85,0 | 0,206 | 51,3 |
| 2,5 g/l | 39,7 | 52,8 | 55,4 | 45,3 | 172,6 | 0,107 | 1,1 |
| 30 g/l | 16,3 | 27,9 | 29,1 | 19,1 | 82,5 | 0,179 | 52,7 |
| 100 g/l | 17,6 | 28,1 | 29,4 | 18,8 | 86,3 | 0,176 | 50,6 |

## Exemple 12 - Acides gras monoéniques isomères cis

On a testé une solution aqueuse de sels sodiques d'acide undécylénique, à des concentrations variant de 10 à 30 g/l Les résultats rassemblés sur le tableau 11 montrent que le pourcentage d'activité varie de 15 à 38 %.

TABLEAU 11

| LOT | % d'oedème | | | | Aire sous la courbe | | % d'activité |
|---|---|---|---|---|---|---|---|
| | t1 | t2 | t3 | t4 | m | CV | |
| Témoin | 30,6 | 50,7 | 62,4 | 59,4 | 173,4 | 0,036 | |
| Al 12 | 15,2 | 25,0 | 33,9 | 32,1 | 90,1 | 0,094 | 48,0 |
| 10 g/l | 20,5 | 43,2 | 56,9 | 52,7 | 146,3 | 0,056 | 15,6 |
| 20 g/l | 16,0 | 32,0 | 39,8 | 38,6 | 107,1 | 0,119 | 38,2 |
| 30 g/l | 18,1 | 34,7 | 45,5 | 39,3 | 117,7 | 0,143 | 32,1 |

Des résultats similaires ont été obtenus avec des solutions aqueuses d'acide érucique.

On a testé une solution aqueuse de sels sodiques d'acide palmitoléique, à des concentrations variant de 10 à 30 g/l Les résultats rassemblés sur le tableau 12 montrent que le pourcentage d'activité varie de 44 à 72 %.

TABLEAU 12

| LOT | % d'oedème | | | | Aire sous la courbe | | % d'activité |
|---|---|---|---|---|---|---|---|
| | t1 | t2 | t3 | t4 | m | CV | |
| Témoin | 39,9 | 57,7 | 59,2 | 52,7 | 183,0 | 0,037 | |
| AI 12 | 10,0 | 18,6 | 20,0 | 16,4 | 56,6 | 0,201 | 69,1 |
| 10 g/l | 18,1 | 31,6 | 36,4 | 32,4 | 102,0 | 0,097 | 44,3 |
| 20 g/l | 9,1 | 19,7 | 24,6 | 21,0 | 64,0 | 0,137 | 65,0 |
| 30 g/l | 7,3 | 15,5 | 19,5 | 18,4 | 51,7 | 0,204 | 71,7 |

Exemple 13 - Mélanges d'acides gras.

Des solutions aqueuses de sels sodiques d'acides gras obtenues à partir d'un mélange de 11 % d'acide octanoïque, de 10 % d'acide décanoïque, de 68 % d'acide laurique, de 9,5 % d'acide myristique et de 1 % d'acide palmitique ont été testées à des concentrations variant de 5 à 20 g/l. Le pourcentage d'activité varie de 19 à 40 %. L'utilisation de solutions plus concentrées exige une adaptation de la posologie.

Par ailleurs on a testé une solution aqueuse de sels potassiques d'acides gras à 30 g/l obtenue à partir d'un mélange de 70 % d'acide oléique, 5 % d'acide palmitique, 6 % d'acide palmitoléïque, 12 % d'acide linoléique, 2 % d'acide arachidique, 2 % d'acide myristique, 2 % d'acide stéarique, 1 % d'acide linolénique. Le pourcentage d'activité est de 60 %.

Exemple 14 - Acide dihydroxystéarique

On a testé une solution aqueuse de sels sodiques d'acide dihydroxystéarique, à des concentrations variant de 10 à 30 g/l. Les résultats rassemblés sur le tableau 13 montrent que le pourcentage d'activité varie de 44 à 57 %.

TABLEAU 13

| LOT | % d'oedème | | | | Aire sous la courbe | | % d'activité |
|---|---|---|---|---|---|---|---|
| | t1 | t2 | t3 | t4 | m | CV | |
| Témoin | 16,1 | 41,9 | 54,2 | 51,6 | 139,0 | 0,079 | |
| AI 12 | 12,3 | 26,9 | 34,5 | 33,3 | 90,2 | 0,146 | 35,1 |
| 10 g/l | 11,7 | 24,4 | 27,8 | 24,9 | 75,7 | 0,198 | 45,5 |
| 20 g/l | 10,7 | 24,7 | 28,9 | 25,2 | 77,2 | 0,195 | 44,5 |
| 30 g/l | 6,7 | 19,0 | 22,9 | 22,4 | 60,1 | 0,147 | 56,8 |

Exemple 15 - Acide hydroxystéarique

On a testé une solution aqueuse de sels sodiques d'acide hydroxystéarique, à des concentrations variant de 10 à 20 g/l Les résultats rassemblés sur le tableau 14 montrent que le pourcentage d'activité varie de 25 à 35 %.

8

TABLEAU 14

| LOT | % d'oedème | | | | Aire sous la courbe | | % d'activité |
|---|---|---|---|---|---|---|---|
| | t1 | t2 | t3 | t4 | m | CV | |
| Témoin | 26,7 | 51,0 | 62,5 | 52,9 | 166,7 | 0,075 | |
| Al 12 | 14,6 | 26,2 | 32,7 | 28,1 | 87,2 | 0,084 | 47,7 |
| 10 g/l | 19,9 | 35,9 | 45,3 | 45,7 | 124,1 | 0,145 | 25,6 |
| 20 g/l | 15,3 | 31,6 | 41,1 | 42,5 | 109,2 | 0,144 | 34,5 |

Exemple 16 - Acide époxystéarique

On a testé une solution aqueuse de sels sodiques d'acide époxystéarique, à des concentrations variant de 10 à 30 g/l Les résultats rassemblés sur le tableau 15 montrent que le pourcentage d'activité varie de 23 à 70 %.

TABLEAU 15

| LOT | % d'oedème | | | | Aire sous la courbe | | % d'activité |
|---|---|---|---|---|---|---|---|
| | t1 | t2 | t3 | t4 | m | CV | |
| Témoin | 22,7 | 50,4 | 62,6 | 59,7 | 165,4 | 0,053 | |
| Al 13 | 14,3 | 29,3 | 39,7 | 37,1 | 102,3 | 0,173 | 38,1 |
| 10 g/l | 18,7 | 38,9 | 45,9 | 44,9 | 126,5 | 0,087 | 23,5 |
| 20 g/l | 15,5 | 30,6 | 36,5 | 36,4 | 102,1 | 0,190 | 38,3 |
| 30 g/l | 6,2 | 13,2 | 21,0 | 20,3 | 50,7 | 0,156 | 69,3 |

Exemple 17 - Acides acétyléniques

On a testé une solution aqueuse de sels sodiques d'acide stéarolique à des concentrations variant de 10 à 30 g/l. Les résultats rassemblés dans le tableau 16 montrent que le pourcentage d'activité varie de 23 à 62 %

TABLEAU 16

| LOT | % d'oedème | | | | Aire sous la courbe | | % d'activité |
|---|---|---|---|---|---|---|---|
| | t1 | t2 | t3 | t4 | m | CV | |
| Témoin | 26,2 | 53,2 | 58,2 | 55,1 | 165,1 | 0,056 | |
| Al 13 | 10,4 | 22,2 | 29,8 | 27,5 | 76,2 | 0,096 | 53,8 |
| 10 g/l | 16,4 | 39,9 | 48,0 | 45,2 | 126,7 | 0,080 | 23,3 |
| 20 g/l | 13,1 | 29,1 | 35,9 | 36,3 | 96,7 | 0,125 | 41,4 |
| 30 g/l | 10,6 | 18,7 | 21,9 | 23,0 | 62,8 | 0,132 | 62,0 |

Les compositions selon l'invention sont administrées à l'homme ou à l'animal pour traiter les états inflammatoires aigus ou chroniques tels que les états rhumatoïdes ou arthrosiques ou la spondylarthrite ankylosante. Elles peuvent efficacement être associées à un ou plusieurs anti-inflammatoires stéroïdien et/ou non stéroïdien, comme le montrent les exemples suivants.

Dans ces exemples, la référence DF 21 correspond à un mélange 2/3 suif -1/3 coprah, (30 g/l), la référence DF 35 à de l'huile de saumon (30 g/l)et la référence DF 78 à un mélange 50 % de suif, 25 % d'huile de coprah et 25 % d'huile d'olive (30 g/l).

Dans les exemples 18 à 21 , le pourcentage d'oedème a été obtenu grâce à l'unique mesure de l'oedème à T + 3 h après l'administration de la carragénine.

Exemple 18 - Association avec l'aspirine

| TRAITEMENTS | | % d'oedème | % de protection |
|---|---|---|---|
| Témoins : | sérum physiologique<br>0,9 ml/250 g/IM<br>T - 18 h | 52 % | |
| DF 21 | 0,9 ml/250 g/IM<br>T - 18 h | 34 % | 33 % |
| Aspirine | 72 mg/Kg<br>1 ml / 100 g / per os<br>T - 1 h | 43 % | 20 % |
| Association des traitements DF 21 et aspirine - Posologies identiques aux deux lots précédents | | 25 % | 52 % |
| Aspirine | 175 mg/Kg<br>1 l / 100 g/ per os<br>T - 1 h | 36 % | 38 % |

Exemple 19 - Association avec l'aspirine

| TRAITEMENTS | | % d'oedème | % de protection |
|---|---|---|---|
| Témoins : | sérum physiologique<br>0,9 ml/250 g/IM<br>T - 18 h | 53 % | |
| DF 78 | 0,9 ml/250 g/IM<br>T - 18 h | 44 % | 15 % |
| Aspirine | 72 mg/Kg<br>1 ml / 100 g / per os<br>T 1 h | 43 % | 20 % |
| Association des traitements DF 78 et aspirine - Posologies identiques aux deux lots précédents | | 34 % | 37 % |

### Exemple 20 - Association avec la phénylbutazone

| TRAITEMENTS | | % d'oedème | % de protection |
|---|---|---|---|
| Témoins : | sérum physiologique | | |
| | 0,9 ml/250 g/IM | 52 % | |
| | T - 18 h | | |
| DF 78 | 0,9 ml/250 g/IM | 44 % | 13 % |
| | T - 18 h | | |
| Phénylbutazone : | 25 mg/Kg | | |
| | 1 ml / 100 g / per os | 36 % | 31 % |
| | T - 1 h | | |
| Association des traitements DF 78 et phénylbutazone - Posologies identiques aux deux lots précédents | | 30 % | 46,7 % |
| Phénylbutazone: 60 mg/Kg | | | |
| | 1 m l / 100 g / per os | 31 % | 45 % |
| | T - 1 h | | |

### Exemple 21 - Association avec la dexaméthasone, antiinflammatoire stéroïdien)

| TRAITEMENTS | | % d'oedème | % de protection |
|---|---|---|---|
| Témoins : | sérum physiologique | | |
| | 0,9 ml/250 g/IM | 51 % | |
| | T - 18 h | | |
| DF 35 | 0,9 ml/250 g/IM | 40 % | 21 % |
| | T - 18 h | | |
| Dexaméthazone : | 0,1 mg/Kg | | |
| | 1 ml / 100 g / per os | 31 % | 42 % |
| | T - 1 h | | |
| Association des traitements DF 35 et Dexaméthazone Posologies identiques aux deux lots précédents | | 24 % | 60 % |

Dans les exemples 22 à 24, le pourcentage d'activité a été calculé à partir de l'aire sous la courbe obtenue par les mesures de l'oedème à T + 1 h, T + 2 h, T + 3 h et T + 4 h après l'administration de la carragénine.

Exemple 22 - Association avec l'aspirine

| TRAITEMENTS | | % d'oedème | | | | Aire sous la courbe | % d'activité |
|---|---|---|---|---|---|---|---|
| | | 1 h | 2 h | 3 h | 4 h | (avec IC) | |
| Témoins : | sérum physiologique 0,9 ml/250 g/IM T - 18 h | 43,3 | 55,5 | 55,8 | 45,1 | 155,6 ± 12,8 | |
| DF 35 | 0,9 ml/250 g/IM T - 18 h | 30,8 | 42,6 | 42,6 | 34,1 | 118,6 ± 23,3 | 23,8 % |
| Aspirine | 72 mg/Kg 1 ml / 100 g /per os T - 1 h | 25,0 | 34,6 | 35,9 | 26,8 | 97,6 ± 24,1 | 37,3 % |
| Association des traitements DF 35 et aspirine - Posologies identiques aux deux lots précédents | | 15,4 | 21,4 | 26,7 | 20,9 | 69,0 ± 13 | 55,7 % |

Exemple 23 - Association avec la phénylbutazone

| TRAITEMENTS | | % d'oedème | | | | Aire sous la courbe | % d'activité |
|---|---|---|---|---|---|---|---|
| | | 1 h | 2 h | 3 h | 4 h | (avec IC) | |
| Témoins: | sérum physiologique 0,9 ml/250 g / IP/T-3h 1 ml/100 g/ per os/T-1h | 21,48 | 49,6 | 56,1 | 54,7 | 155,3 ± 0,045 | |
| DF 21 | 0,9 ml/250 g/IP T - 3h | 12,5 | 44,0 | 52,3 | 51,2 | 134,7 ± 0,062 | 13,3 % |
| Phénylbutazone | 12,5 mg/Kg 1 ml / 100 g / per os T - 1 h | 12,0 | 39,5 | 42,0 | 45,2 | 116,4 ± 0,059 | 25,0 % |
| Association des traitements DF 21 et Phénylbutazone Posologies identiques aux deux lots précédents | | 10,3 | 32,9 | 38,8 | 36,5 | 100,9 ± 0,080 | 35,0 % |

Exemple 24 - Association avec le kétoprofène

| TRAITEMENTS | | % d'oedème | | | | Aire sous la courbe | % d'activité |
|---|---|---|---|---|---|---|---|
| | | 1 h | 2 h | 3 h | 4 h | (avec IC) | |
| Témoins: | sérum physiologique 0,9 ml/250 g / IP/T-3h 1 ml/100 g/ per os/T-1h | 28,9 | 53,6 | 59,7 | 58,6 | 171,4 ± 0,039 | |
| DF 21 | 0,9 ml/250 g/IP T - 3h | 20,5 | 42,4 | 52,5 | 50,9 | 142,1 ± 0,089 | 17,1 % |
| Kétoprofène | 4,5 mg/Kg 1 ml / 100 g / per os T - 1 h | 15,7 | 38,7 | 45,5 | 43,9 | 122,1 ± 0,073 | 28,8 % |
| Association des traitements DF 21et Kétoprofène. Posologies identiques aux deux lots précédents | | 14,6 | 33,8 | 36,3 | 35,3 | 102,4 ± 0,086 | 40,3 % |

Ces exemples démontrent l'intérêt thérapeutique de l'association d'une composition selon l'invention avec des anti-inflammatoires stéroïdiens (AIS) ou non stéroïdiens (AINS).

On peut noter en particulier :

- que, pour un même effet antiinflammatoire, il y a administration de doses d'AIS ou d'AINS beaucoup plus faibles.
- que la diminution des doses administrées d'AIS ou d'AINS entraîne une diminution de la toxicité induite par le traitement.

**Revendications**

**1.** Compositions douées de propriétés anti-inflammatoires, caractérisées en ce qu'elles renferment, à titre de principe actif principal, dans un support pharmaceutiquement acceptable, un ou plusieurs sels d'un ou plusieurs acides gras présentant la formule générale :

$$CH_3 - C_x..H_y..O_z - COO^- X^+$$

dans laquelle x = 2 à 30, y = 2 à 60, z =0 à 6 et $X^+$ est le cation de de la base utilisée.

**2** Compositions douées de propriétés anti-inflammatoires selon la revendication 1, caractérisées en ce que le ou les acides gras sont choisis parmi ceux contenus dans les substances naturelles, et notamment les huiles et graisses d'origine animale ou végétale.

**3.** Compositions douées de propriétés anti-inflammatoires selon la revendication 1 ou la revendication 2, caractérisées en ce que la base est choisie parmi la soude ou la potasse.

**4.** Compositions douées de propriétés anti-inflammatoires selon l'une des revendications 1 à 3, caractérisées en ce que les acides gras sont saturés, contiennent un nombre pair ou impair d'atomes de carbone et sont éventuellement ramifiés ; ils présentent alors les formes iso et antéiso.

**5.** Compositions douées de propriétés anti-inflammatoires selon l'une des revendications 1 à 3, caractérisées en ce que les acides gras sont monoéniques et sont choisis, pour un même acide, parmi les différents isomères, notamment les acides monoéniques à double liaison en 9, les acides monoéniques à double liaison en n-9 et les acides monoéniques à double liaison cis ou trans.

**6.** Compositions douées de propriétés anti-inflammatoires selon l'une des revendications 1 à 3, caractérisées en ce que les acides gras sont polyéniques et sont choisis, pour un même acide, parmi les différents isomères, notamment les acides polyéniques à méthylènes centraux, les acides polyéniques à doubles liaisons en forme cis et les acides polyéniques à doubles liaisons conjuguées ou écartées.

**7.** Compositions douées de propriétés anti-inflammatoires selon l'une des revendications 1 à 3, caractérisées en ce que les acides gras sont choisis parmi les acides acétyléniques.

**8.** Compositions douées de propriétés anti-inflammatoires selon l'une des revendications 1 à 3, caractérisées en ce que les acides gras sont choisis parmi les acides cycliques.

**9.** Compositions douées de propriétés anti-inflammatoires selon l l'une des revendications 1 à 3, caractérisées en ce que les acides gras sont choisis parmi les hydroxyacides et les cétoacides.

**10.** Compositions douées de propriétés anti-inflammatoires selon l'une quelconque des revendications 1 à 9 caractérisées en ce que le ou les sels d'acides gras sont associés à un ou plusieurs anti-inflammatoires stéroïdien ou non stéroïdien.

**11.** Compositions douées de propriétés anti-inflammatoires selon l'une quelconque des revendications 1 à 10, prévues pour application par voie parentérale, caractérisées en ce qu'elles renferment des concentrations de produit actif entre 2,5 et 120 g.l

**12.** Compositions selon la revendication 11, caractérisées en ce qu'elles renferment des concentrations de produit actif entre 10 et 40 g.l

**13.** Compositions douées de propriétés anti-inflammatoires selon l'une quelconque des revendications 1 à 10, prévues pour application par voie orale.

**14.** Compositions douées de propriétés anti-inflammatoires selon l'une quelconque des revendications 1 à 10, prévues pour application par voie rectale.

**15.** Compositions douées de propriétés anti-inflammatoires selon l'une quelconque des revendications 1 à 10, prévues pour application par voie diathélique.

**16.** Compositions douées de propriétés anti-inflammatoires selon l'une quelconque des revendications 1 à 10, prévues pour application par voie dermique et intradermique.

**Fig.1**

**Fig. 2**

**Fig.3**

**Office européen des brevets**

# RAPPORT DE RECHERCHE EUROPEENNE

Numero de la demande
**EP 94 42 0212**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP-A-0 107 188 (EISAI CO. LTD) 2 Mai 1984<br>---  | | A61K31/20 |
| A | WO-A-90 09977 (LABORATOIRES NATURA MEDICA) 7 Septembre 1990<br>----- | | |

**DOMAINES TECHNIQUES RECHERCHES (Int.Cl.6)**

A61K

Le présent rapport a été établi pour toutes les revendications

| Lieu de la recherche | Date d'achèvement de la recherche | Examinateur |
|---|---|---|
| LA HAYE | 8 Septembre 1994 | Klaver, T |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)